# EUROPEAN PATENT SPECIFICATION

(11) **EP 3 196 290 B1**
(45) Date of publication and mention of the grant of the patent: **08.07.2020**
(21) Application number: 15834937.3
(22) Date of filing: 27.08.2015
(51) Int. Cl.: A01N 1/02, A61B 10/00, C12M 1/26, C12M 1/24, C12N 5/07

(54) **TOOL FOR CRYOPRESERVING COLLECTED BIOLOGICAL TISSUE, AND METHOD FOR CRYOPRESERVING TISSUE FRAGMENT**
WERKZEUG ZUR KRYOKONSERVIERUNG VON GESAMMELTEM BIOLOGISCHEM GEWEBE UND VERFAHREN ZUR KRYOKONSERVIERUNG VON GEWEBEFRAGMENTEN
OUTIL DE CRYOPRÉSERVATION DE TISSUS BIOLOGIQUES COLLECTÉS ET PROCÉDÉ DE CRYOPRÉSERVATION DE FRAGMENT DE TISSUS

(30) Priority: 29.08.2014 JP 2014175476
(43) Date of publication of application: 26.07.2017
(73) Proprietor: Kitazato Corporation, Fuji-shi, Shizuoka 416-0907 (JP); St. Marianna University School of Medicine, Kawasaki-shi Kanagawa 216-8511 (JP)
(72) Inventor: SUZUKI Nao, Kawasaki-shi Kanagawa 216-8511 (JP); INOUE Futoshi, Fuji-shi Shizuoka 416-0907 (JP)
(74) Representative: Casalonga
(86) International application number: PCT/JP2015/074233
(87) International publication number: WO 2016/031916

(56) References cited:
- WO-A1-2006/059626
- WO-A1-2006/059626
- JP-A- 2003 267 471
- JP-A- 2007 302 256
- JP-A- 2008 222 640
- JP-A- 2009 148 221
- JP-A- 2009 148 221
- JP-A- 2012 219 017
- JP-A- 2012 219 017
- JP-U- H0 735 371
- JP-Y1- S 487 581
- US-A1- 2011 120 148
- MK Alliance Inc: "Overwraps", TotipotentSC , 18 October 2011 (2011-10-18), XP002779232, Retrieved from the Internet: URL:http://www.totipotentsc.com/overwraps. php [retrieved on 2018-03-16]

## Description

### TECHNICAL FIELD

The present invention relates to a collected living tissue cryopreserving appliance to be used in cryopreserving living tissues such as mammalian ova and a tissue piece cryopreserving method.

### BACKGROUND ART

Living cells (for example, sperm, ovum, and the like) of mammals are cryopreserved. The cryopreservation of the living cells enables the preservation of hereditary resources of a specified system and kind, is effective for maintaining animal species standing on the verge of extinction, and is useful for sterile treatment.

An ovary freezing method is disclosed in Japanese Patent Application Laid-Open Publication No. 2003-284546 (patent document 1). The ovary freezing method is characterized in that a reproductive tissue including at least an ovary taken out from a female individual of an impersonal mammal is preserved at 15 to zero degrees C and thereafter frozen by a vitrification method.

As a method for cryopreserving mammalian embryos, as disclosed in Japanese Patent Application Laid-Open Publication No. 2000-189155 (patent document 2), there is proposed a method of sticking mammalian embryos or ova to the inner surface of the sterilized cryopreserving container such as the freezing straw, the freezing vial or the freezing tube by using a minimum amount of the vitrification solution sufficient for encapsulating the mammalian embryos or the ova therein, sealing the cryopreserving container, and bringing the cryopreserving container into contact with liquid nitrogen to rapidly cool the cryopreserving container. In the disclosed thawing method, the cryopreserving container preserved in the above-described method is taken out from the liquid nitrogen and one end thereof is opened to directly inject a diluted liquid having the temperature of 33 to 39 degrees C into the container. In this way, the vitrification solution was diluted to thaw the frozen mammalian embryos or ova. This method has an excellent effect of eliminating a possibility that the mammalian embryos or ova are infected with diseases through viruses or bacteria and allowing them to be cryopreserved at a high survival rate and thawed by diluting the vitrification solution.

The present applicant proposed the tissue piece cryopreserving plate as disclosed in Japanese Patent Application Laid-Open Publication No. 2008-222640 (patent document 3). The tissue piece cryopreserving plate 4 is the plate-shaped metal member which has the width equal to or a little larger than the predetermined width of the cutting portion-specifying open portion 22, is extended at the predetermined length, allows the tissue piece cut off from a living tissue by using the tissue cutting position-specifying tool 10 to be placed thereon, and has a large number holes.

The present applicant proposed the cryopreserving tool as disclosed in Japanese Patent Application Laid-Open Publication No. 2012-219017 (patent document 4). The cryopreserving tool of the patent document 4 is used to freeze a biological specimen such as tissue pieces, cells, and the like by means of the vitrification freezing method (including rapid vitrification freezing method and super-rapid vitrification freezing method). The cryopreserving tool has the specimen placing part including the stainless sheet and the holding part fixed to the proximal end portion of the specimen placing part. The cryopreserving tool does not have a through-hole in the region of the specimen placing part where the biological specimen is to be placed, but the stainless sheet is used as the specimen placing part.

### PRIOR ART DOCUMENTS

### PATENT DOCUMENTS

Patent document 1: Japanese Patent Application Laid-Open Publication No. 2003-284546
Patent document 2: Japanese Patent Application Laid-Open Publication No. 2000-189155
Patent document 3: Japanese Patent Application Laid-Open Publication No. 2008-222640 (US2010-003662A)
Patent document 4: Japanese Patent Application Laid-Open Publication No. 2012-219017 US 2011/120148 discloses a container holder and container, lacking an accommodating bag as well as specific container features such as container transparency, a tissue piece placing member, a fixing member and a sealing member, as well as further container holder features such as opposing curved portions with greater diameter for receiving an accommodating bag with a container and rounded edges for bag protection during bag/container insertion.

### SUMMARY OF THE INVENTION

The invention is defined in the claims.

### PROBLEM TO BE SOLVED BY THE INVENTION

The tissue piece cryopreserving plate and the cryopreserving tool disclosed in the patent documents 3 and 4 respectively are effective for freezing the tissue piece by vitrification. Both of the tissue piece cryopreserving plate and the cryopreserving tool are based on the premise that the tissue piece is directly brought into contact with the liquid nitrogen which is the cooling medium and that the container accommodating the tissue piece is directly brought into contact with the cooling medium (liquid nitrogen) during the cryopreservation of the tissue piece. But a long-term contact between the container and the cooling medium (liquid nitrogen) may cause the container to deteriorate and the cooling medium to flow thereinto. The direct contact of the tissue piece with the cooling medium is effective for freezing the tissue piece by vitrification. But if possible, the direct contact of such a substance with cells should be avoided. The tissue piece cryopreserving plate and the cryopreserving tool disclosed in the patent documents 3 and 4 respectively are sealed inside the container with the liquid nitrogen attaching to the living tissue and the tissue piece placing portion. After the living tissue is cryopreserved, the container is opened in a normal temperature environment when the living tissue is used. When the container is opened without gradually performing an opening operation, an inner pressure caused by the liquid nitrogen vaporized inside the container may scatter the living tissue piece placed on the tissue piece placing portion. Therefore it is necessary to carefully perform the opening operation.

It is an object of the present invention to provide a collected living tissue cryopreserving appliance which allows a freezing operation without bringing a collected living tissue piece into contact with a cooling medium, prevents the living tissue piece from contacting the cooling medium even when it is cryopreserved for a long time, and prevents the living tissue piece from scattering when an operation of opening a container is performed after the living tissue piece is cryopreserved and a tissue piece cryopreserving method.

### MEANS FOR SOLVING THE PROBLEMS

The above-described object is achieved by a collected living tissue cryopreserving appliance described below.

The collected living tissue cryopreserving appliance is used to cryopreserve a collected living tissue piece in a sealed state. The collected living tissue cryopreserving appliance is composed of a collected tissue accommodating container capable of accommodating the collected living tissue in a sealed state, a resin transparent accommodating bag which is capable of accommodating the collected tissue accommodating container therein, can be sealed by heat-sealing the resin transparent accommodating bag after accommodating the collected tissue accommodating container therein, and is resistant to liquid nitrogen, and a long and narrow container holding member having a container holding part, disposed at a side thereof, for holding the collected tissue accommodating container accommodated inside the accommodating bag.

The collected tissue accommodating container is composed of a transparent container body having an open portion and a lid member which can be removably mounted on the open portion of the transparent container body and can be mounted thereon in an airtight state. The lid member has a lid body part, a tissue piece placing member where the living tissue piece is mountable, a fixing member for fixing the tissue piece placing member to the lid body part, and a sealing member, formed on an inner surface of the lid body part, for sealing the open portion of the transparent container body.

The above-described object is also achieved by a tissue piece cryopreserving method using the above-described collected living tissue cryopreserving appliance described below.

The tissue piece cryopreserving method includes a tissue piece immersing step of immersing a tissue piece collected from a living tissue in a vitrification solution; a tissue piece placing step of taking out the tissue piece from the vitrification solution and placing the tissue piece on the tissue piece placing member of the lid member; a tissue piece sealing step of inserting the tissue piece placing member on which the tissue piece has been placed into the transparent container body without bringing the tissue piece placing member into contact with a cooling medium, mounting the lid member on the container body, and sealing the collected tissue accommodating container; an accommodating bag sealing step of accommodating the sealed collected tissue accommodating container inside the accommodating bag and thereafter heat-sealing an open portion of the accommodating bag; a container body mounting step of mounting the accommodating bag which has accommodated the collected tissue accommodating container therein and has been sealed on the container holding part of the container holding member; and an accommodating step of accommodating the container holding member holding the packaged container body inside a cooling medium tank.

### BRIEF DESCRIPTION OF THE DRAWINGS

Fig. 1 is a front view of one embodiment of a collected living tissue cryopreserving appliance of the present invention.
Fig. 2 is a left side view of the collected living tissue cryopreserving appliance shown in Fig. 1.
Fig. 3 is a front view of a collected tissue accommodating container for use in the collected living tissue cryopreserving appliance shown in Fig. 1.
Fig. 4 is a left side view of the collected tissue accommodating container shown in Fig. 3.
Fig. 5 is a sectional view taken along a line A-A of Fig. 3.
Fig. 6 is a sectional view taken along a line B-B of Fig. 4.
Fig. 7 is a front view of a lid member having a tissue piece placing member for use in the collected tissue accommodating container shown in Fig. 3.
Fig. 8 is a left side view of the lid member having the tissue piece placing member shown in Fig. 7.
Fig. 9 is a bottom view of the lid member having the tissue piece placing member shown in Fig. 7.
Fig. 10 is a front view of a container body for use in the collected tissue accommodating container shown in Fig. 3.
Fig. 11 is a front view of a lid member having a tissue piece placing member for use in a collected living tissue cryopreserving appliance of another embodiment of the present invention.
Fig. 12 is a front view of a lid member having a tissue piece placing member for use in a collected living tissue cryopreserving appliance of still another embodiment of the present invention.
Fig. 13 is an explanatory view for explaining the action of the collected living tissue cryopreserving appliance of the present invention.
Fig. 14 is a front view of a container holding member for use in the collected living tissue cryopreserving appliance shown in Fig. 1.
Fig. 15 is a left side view of the container holding member for use in the collected living tissue cryopreserving appliance shown in Fig. 1.

### MODE FOR CARRYING OUT THE INVENTION

The collected living tissue cryopreserving appliance of the present invention and the tissue piece cryopreserving method thereof are described below by using the embodiments shown in the drawings.

A collected living tissue cryopreserving appliance 1 of the present invention is used to cryopreserve a collected living tissue piece 12 in a sealed state. The collected living tissue cryopreserving appliance 1 is composed of a collected tissue accommodating container 2 capable of accommodating the collected living tissue in a sealed state, a resin transparent accommodating bag 5 which is capable of accommodating the collected tissue accommodating container 2 therein, can be sealed by heat-sealing the resin transparent accommodating bag after accommodating the collected tissue accommodating container 2, and is resistant to liquid nitrogen, and a long and narrow container holding member 6 having a container holding part 62, disposed at a side thereof, for holding the collected tissue accommodating container 2 accommodated inside the accommodating bag 5.

The collected tissue accommodating container 2 is composed of a bottomed cylindrical container body 3 having an open portion and a lid member 4 which can be removably mounted on the open portion of the bottomed cylindrical container body and can be mounted thereon in an airtight state. The lid member 4 has a lid body part 42, a tissue piece placing member 41, a fixing member for fixing the tissue piece placing member 41 to the lid body part 42, and a sealing member 48, formed on an inner surface of the lid body part 42, for sealing the open portion of the transparent container body 3.

The collected living tissue cryopreserving appliance 1 of this embodiment is composed of the collected tissue accommodating container 2, the accommodating bag 5 for accommodating the collected tissue accommodating container 2 therein, and the long and narrow container holding member 6 having the container holding part 62, disposed at the side thereof, for holding the collected tissue accommodating container 2 accommodated inside the accommodating bag 5.

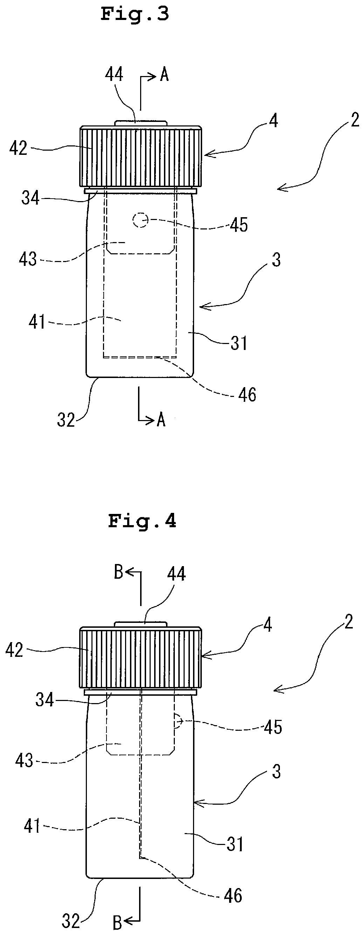

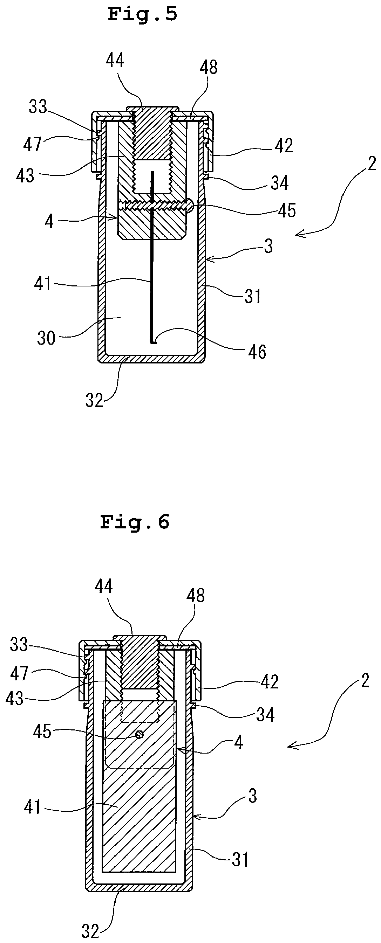

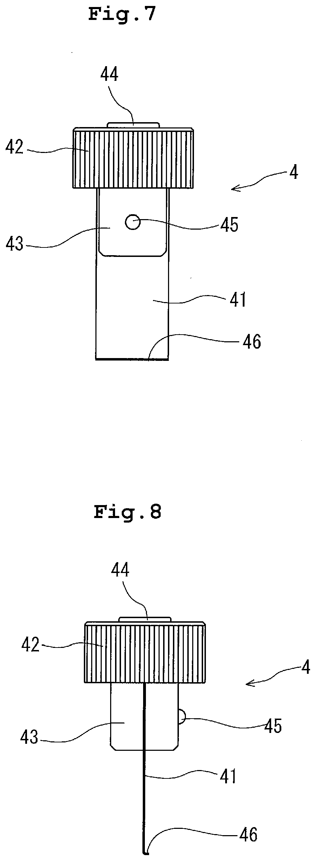

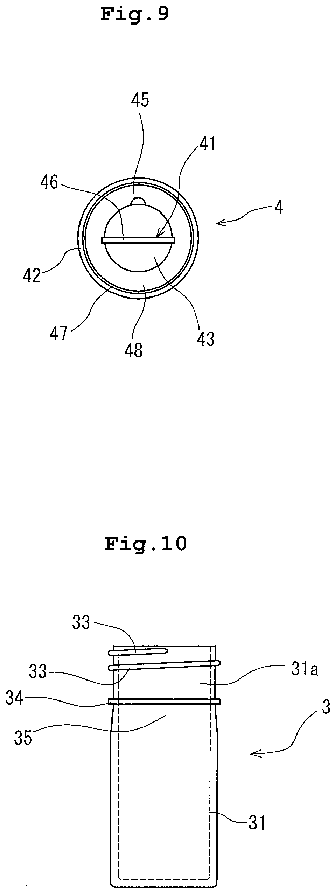

As shown in Figs. 3 through 10, the collected tissue accommodating container 2 is composed of the bottomed cylindrical container body 3 having the open portion and the lid member 4 which can be removably mounted on the open portion of the bottomed cylindrical container body and can be mounted thereon in an airtight state. As shown in Fig. 10, the bottomed cylindrical container body 3 used in this embodiment is bottomed and substantially cylindrical. It is preferable that the container body 3 is transparent and particularly preferable that the container body is colorless and transparent.

The container body 3 has a cylindrical body part 31 having almost the same outer diameter in its vertical direction, a bottom part 32, an upper open part, and an inner accommodating part 30. A container body side threaded engaging portion 33 is formed on an outer surface of the upper open part of the container body. In the container body 3, an upper portion of the body part 31 is formed as a small-diameter portion 31a having a little smaller diameter than the remaining portion thereof. The container body side threaded engaging portion 33 is formed on the small-diameter portion. A flange portion 34 is formed at a proximal end portion, of the small-diameter portion 31a, which is located lower than the position of the container body side threaded engaging portion 33 at a predetermined length. The container body 3 has a tapered portion 35 whose diameter increases from the flange portion 34 to a proximal end thereof.

The lid member 4 has the lid body part 42, the tissue piece placing member 41, the fixing member for fixing the tissue piece placing member 41 to an inner side of the lid body part 42, and the sealing member 48, provided to the inner surface of the lid body part 42, for sealing the open portion of the transparent container body 3.

The lid body part 42 is a short cylindrical member having an upper surface closed portion and has a lid member side threaded engaging portion 47, formed on an inner surface of an annular portion thereof, which is capable of threadedly engaging the container body side threaded engaging portion 33. The lid member side threaded engaging portion 47 is formed of a spiral rib. The sealing member 48 is accommodated on the inner surface of the upper surface closed portion. In this embodiment, the upper surface closed portion and the sealing member 48 have an opening respectively for inserting a fixing tool 44 described later thereinto.

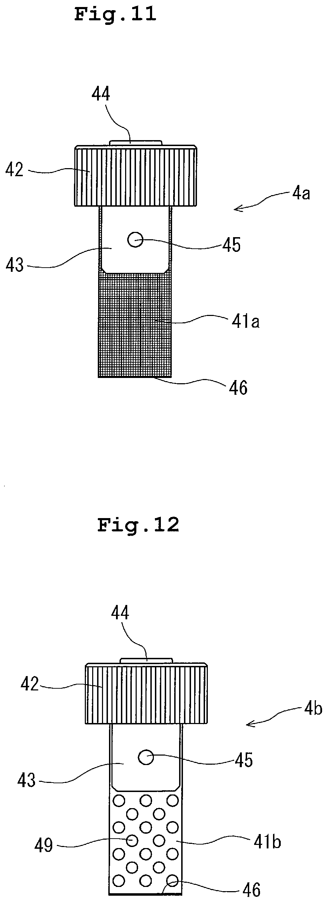

The tissue piece placing member 41 of the lid member 4 is formed of a plate-shaped member extended at a predetermined length. As the plate-shaped member, it is preferable to use a metallic plate-shaped member, a graphite sheet, and the like having a high thermal conductivity. As metals forming the metallic plate-shaped member, it is possible to use any one of metals selected from among copper, copper alloys, aluminum, aluminum alloys, gold, gold alloys, silver, silver alloys, and stainless steel alloys. The tissue piece placing member may be formed like a tissue piece placing member 41a of a lid member 4a of an embodiment shown in Fig. 11. The tissue piece placing member 41a is formed of a metal wire mesh. The tissue piece placing member may be formed like a tissue piece placing member 41b of a lid member 4b of an embodiment shown in Fig. 12. The tissue piece placing member 41b is formed of a metallic plate-shaped member and has a plurality of holes 49.

Each of the tissue piece placing members 41, 41a, and 41b of these embodiments has a front-end bent portion 46. The bent portion 46 prevents the tissue piece from separating (drop) from the tissue piece placing member. The lid member 4 has a holding member 43 for holding a proximal end portion of the tissue piece placing member 41 and the fixing tool 44 for fixing the holding member 43 to the inner surface of the lid body part 42. The holding member 43 has a slit into which the proximal end portion of the tissue piece placing member 41 can be inserted at one end side thereof and a concave part having a female screw portion at the other end side thereof. The tissue piece placing member 41 is inserted into the slit of the holding member 43 and fixed to the holding member 43 by means of a screw member 45 threadedly engaging a side hole of the holding member 43. The fixing tool 44 has a male screw portion threadedly engageable with the female screw portion formed on the concave part of the holding member 43. The fixing tool 44 has a head whose diameter is larger than that of the male screw portion thereof. A peripheral edge of an open portion disposed on an upper surface of the lid body part 42 and a peripheral edge of an open portion of the sealing member 48 are pressurized by the head of the fixing tool 44 and an end portion of the holding member 43.

The holding member 43 is cylindrical and has an outer diameter set smaller than an inner diameter of the open portion of the container body 3. In this embodiment, a head of the screw member 45 attached to the holding member 43 does not contact an inner surface of the container body 3.

In this embodiment, in a state where the lid member 4 is mounted on the container body 3 and the open portion of the container body is sealed with the lid member 4, in other words, in a state where the sealing member 48 of the lid member 4 closely contacts the open portion of the container body 3, a front end of the tissue piece placing member 41 does not contact a bottom surface of the container body 3.

The accommodating bag 5 has a capacity capable of accommodating the collected tissue accommodating container 2 (composed of the container body 3 and the lid member 3). A part of a peripheral edge of the accommodating bag 5 is open to allow the collected tissue accommodating container 2 to be inserted thereinto.

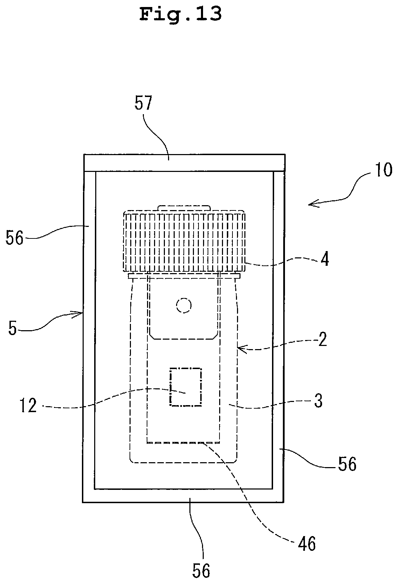

More specifically, the accommodating bag 5 is formed by laminating two rectangular transparent resin films with each other and heat-sealing the two films to each other at three sides 56 thereof. The side of each film not heat-sealed forms the open portion of the accommodating bag. The open portion thereof can be heat-sealed with the accommodating bag 5 accommodating the collected tissue accommodating container 2 therein. As shown in Fig. 13, the open portion of the accommodating bag 5 is formed as a sealed portion 57.

As shown in Fig. 13, the accommodating bag 5 is made of resin resistant to liquid nitrogen and is transparent. It is preferable to form the accommodating bag 5 by laminating two resin films each having an outer layer (for example, high-density polyethylene, polyethylene terephthalate) resistant to the liquid nitrogen and a heat-sealable inner layer (low melting point polyethylene) with each other with the heat-sealable inner layers being opposed to each other and by heat-sealing peripheral edges of the inner layers to each other in the shape of U. It is preferable to form the outer layer of a biaxially oriented film. For example, it is preferable to use a biaxially oriented polyester film (biaxially oriented polyethylene terephthalate film), a biaxially oriented polypropylene film, a biaxially oriented polystyrene film, a biaxially oriented polyamide film, and a high-density biaxially oriented polyethylene film. It is especially preferable that these films are transparent.

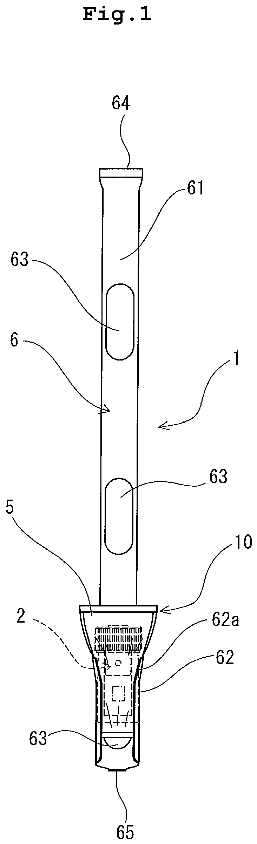

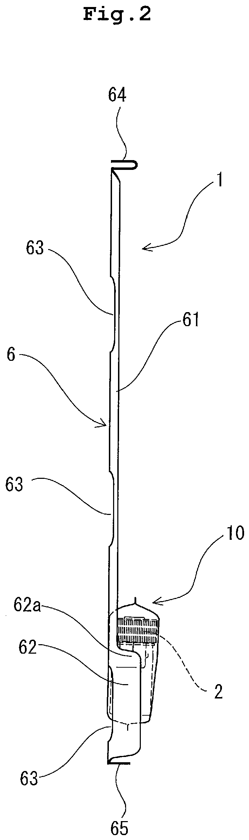

As shown in Figs. 1, 2, 14, and 15, the container holding member 6 is a long and narrow member extended at a predetermined length and has the container holding part 62, disposed at the side thereof, for holding the collected tissue accommodating container 2 accommodated inside the accommodating bag 5.

The container holding member 6 has a long and narrow holding member body 61 axially extended at a predetermined length and curved at its bottom surface, the container holding part 62 provided at a lower portion of the holding member body, and a cooling medium circulating open portion 63. The container holding part 62 of the container holding member 6 of this embodiment is provided in the vicinity of a lower end of the container holding member 6. A plurality of the cooling medium circulating open portions 63 is formed on the holding member body.

The container holding member 6 is subjected to rounding machining so that the container holding member does not have an edge which may damage the accommodating bag 5 where the container holding member 6 is accommodated (mounted). The container holding part 62 of the container holding member 6 is axially extended at a predetermined length and formed of two curved portions opposed to each other. A large-diameter portion 62a is formed between upper portions of the two opposed curved portions of the container holding part 62. The interval between the upper portions of the two opposed curved portions thereof is set larger than other portions thereof and the outer diameter of a lower part of the bottomed cylindrical container body. Therefore it is easy to mount the collected tissue accommodating container 2 accommodated inside the accommodating bag 5 on the container holding part 62. The outer diameter of a part of the container holding part 62 disposed below the large-diameter portion 62a is set equally to or a little smaller than that of the lower part of bottomed cylindrical container body 3 to allow the container holding part 62 to hold the container body.

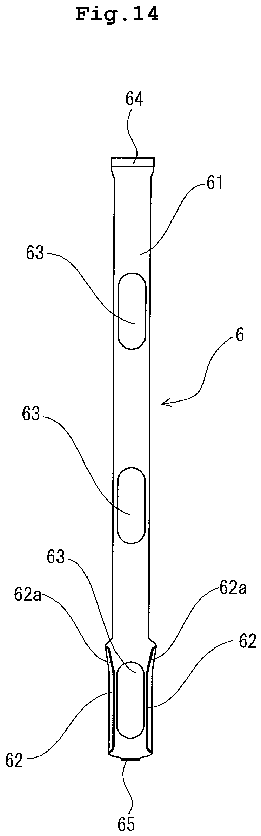

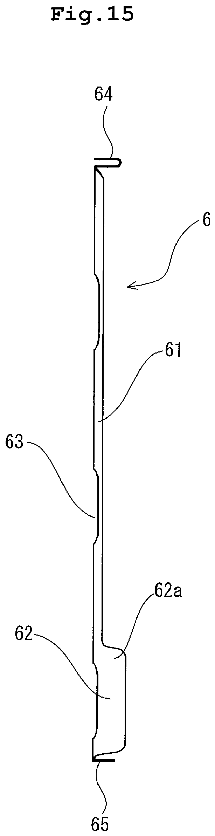

As shown in Figs. 14 and 15, the container holding member 6 of this embodiment further includes a bottom portion 65, disposed at the lower end thereof, which is curved toward the center of the holding member body 61 and a suspension portion 64 disposed at the upper end thereof, which is curved outward from the holding member body 61. The suspension portion 64 is used when the container holding member is suspended on a cylindrical accommodating portion of a canister to be accommodated inside an auxiliary preserving tank.

The collected living tissue cryopreserving appliance 1 of the present invention is very effective as an ovarian tissue piece cryopreserving appliance and can be effectively utilized as a liver tissue piece cryopreserving appliance.

The tissue piece cryopreserving method (method for cryopreserving tissue piece by vitrification) using the collected living tissue cryopreserving appliance 1 is described below.

In the tissue piece cryopreserving method of the present invention, initially, the tissue piece 12 collected from a living tissue to be cryopreserved is prepared. The tissue piece can be collected from the living tissue to be cryopreserved (for example, living organ such as ovary taken out from living body) by using a tissue cutting position-specifying tool as disclosed in Japanese Patent Application Laid-Open Publication No. 2008-222640. The tissue piece may be collected by using a normal scalpel or the like. The tissue piece 12 is collected in a size which can be placed on a surface of the tissue piece placing member 41 of the collected tissue accommodating container 2 held by the container holding member 6.

Thereafter a tissue piece solution immersion step of immersing the collected tissue piece 12 in a vitrification solution is performed.

The tissue piece vitrification solution immersion step is performed by immersing the collected tissue piece 12 in the vitrification solution. It is possible to use vitrification solutions conventionally used. More specifically, it is preferable to use the vitrification solution containing one kind or not less than two kinds of a membrane-permeable cryoprotectant selected from among glycerol, propylene glycol, dimethylsulfoxide (DMSO), ethylene glycol, and butanediol and one kind or not less than two kinds of a membrane-impermeable cryoprotectant selected from among sucrose, trehalose, percoll, polyethylene glycol, polyvinyl pyrrolidone, bovine serum albumin, and ficoll.

It is preferable to perform treatment of immersing the collected tissue piece in an equilibrium liquid before the collected tissue piece is immersed in the vitrification solution. As the equilibrium liquid, a low-concentration solution of the membrane-permeable cryoprotectant is preferable. As the membrane-permeable cryoprotectant, it is possible to use the above-described substances.

After the execution of the step of immersing the tissue piece in the vitrification solution finishes, a tissue piece placing step of taking out the tissue piece from the vitrification solution and placing the tissue piece on the tissue piece placing member 41 of the lid member 4 is performed.

At this step, after the tissue piece is taken out from the vitrification solution, the tissue piece is placed on the tissue piece placing member 41 without bringing the tissue piece into contact with a cooling medium such as the liquid nitrogen. In the operation of placing the tissue piece on the tissue piece placing member 41, the tissue piece is not frozen by vitrification.

Subsequently, a tissue piece sealing step of inserting the tissue piece placing member 41 holding the tissue piece 12 thereon into the transparent container body 3, mounting the lid member 4 on the container body 3, and sealing the collected tissue accommodating container 2 is performed.

At this step, the tissue piece placing member 41 holding the tissue piece 12 thereon is inserted into the container body 3 from the front end of the tissue piece placing member. Thereafter the lid body part 42 is rotated to threadedly engage the threadedly engaging portion 47 of the lid body part 42 and the threadedly engaging portion 33 of the container body 3 with each other. With the progress of the threaded engagement between both threadedly engaging portions, the sealing member 48 of the lid member 4 seals the open portion of the container body 3 with the sealing member 48 in contact with the open portion of the container body. Thereby the open portion of the container body 3 is sealed with the lid member 4. In this state, the front end of the tissue piece placing member 41 does not contact the inner bottom surface of the container body 3.

Subsequently an accommodating bag sealing step of accommodating the collected tissue accommodating container 2 inside the accommodating bag 5 and heat-sealing the open portion of the accommodating bag is performed.

At this step, by initially mounting the lid member 4 on the container body 3 as described above, the collected tissue accommodating container 2 placed in a sealed state is inserted into the accommodating bag 5 from the open portion thereof to accommodate the collected tissue accommodating container inside the accommodating bag. By sealing the open portion of the accommodating bag 5 with a heat sealer, the open portion thereof is formed as the sealed portion 57. Thereby the collected tissue accommodating container 2 is sealed inside the accommodating bag 5.

Subsequently a container body mounting step of mounting the accommodating bag which has accommodated the collected tissue accommodating container 2 therein and has been sealed on the container holding part of the container holding member 6 is performed.

At this step, the accommodating bag which has accommodated the collected tissue accommodating container 2 therein and has been sealed is fitted on the container holding part of the container holding member 6.

An accommodating step of accommodating the container holding member 6 on which a packaged container body 10 has been mounted inside a cooling medium tank is performed. At this step, the container holding member 6 holding the collected tissue accommodating container 2 thereon is supplied into the cooling auxiliary preserving tank in which a predetermined amount of the cooling medium (generally, liquid nitrogen) is filled by injecting the cooling medium thereinto. More specifically, at this step, the container holding member 6 holding the collected tissue accommodating container 2 thereon is supplied into to the cylindrical accommodating portion of the canister accommodated in the tank, or the container holding member 6 is suspended on an upper edge of the cylindrical accommodating portion of the canister.

Thereafter the accommodating bag 5, the collected tissue accommodating container 2, and the tissue piece 12 are cooled inside the cooling auxiliary preserving tank. Because the tissue piece 12 contains the vitrification solution (more specifically, intracellular fluid is replaced with vitrification solution to some extent), the tissue piece is not crystallized but vitrified when the tissue piece is frozen. The tissue piece is preserved inside the tank until it is used.

### INDUSTRIAL APPLICABILITY

The collected living tissue cryopreserving appliance of the present invention has the construction described below.
(1) The collected living tissue cryopreserving appliance is used to cryopreserve the collected living tissue piece in the sealed state. The collected living tissue cryopreserving appliance is composed of the collected tissue accommodating container capable of accommodating the collected living tissue in the sealed state, the resin transparent accommodating bag which is capable of accommodating the collected tissue accommodating container therein, can be sealed by heat-sealing the resin transparent accommodating bag after accommodating the collected tissue accommodating container therein, and is resistant to the liquid nitrogen, and the long and narrow container holding member having the container holding part, disposed at the side thereof, for holding the collected tissue accommodating container accommodated inside the accommodating bag.
   The collected tissue accommodating container is composed of the transparent container body having the open portion and the lid member which can be removably mounted on the open portion of the transparent container body and can be mounted thereon in the airtight state. The lid member has the lid body part, the tissue piece placing member where the living tissue piece is mountable, the fixing member for fixing the tissue piece placing member to the lid body part, and the sealing member, formed on the inner surface of the lid body part, for sealing the open portion of the transparent container body.
   By placing the living tissue piece treated for vitrification on the tissue piece placing member of the lid member, inserting the tissue piece placing member into the container body, and mounting the lid member on the container body, the tissue piece can be placed in a sealed state. Further, by accommodating the sealed collected tissue accommodating container inside the resin transparent accommodating bag and sealing the open portion of the resin transparent accommodating bag, the collected tissue accommodating container itself can be sealed. Thereafter by holding the collected tissue accommodating container sealed inside the accommodating bag on the container holding part of the container holding member, inserting the container holding member into the preserving tank accommodating the cooling medium, and cooling the living tissue piece inside the tank, the living tissue piece is frozen and can be preserved in a frozen state. In the collected living tissue cryopreserving appliance, the collected tissue accommodating container is sealed and entirely sealed by the accommodating bag. Therefore during the cryopreservation of the tissue piece, the cooling medium does not enter the collected tissue accommodating container. Further the collected tissue accommodating container does not contact the cooling medium. Therefore it hardly occurs that the collected tissue accommodating container deteriorates owing to the contact between the collected tissue accommodating container and the cooling medium. The tissue piece does not contact the cooling medium either. Furthermore, the liquid nitrogen is not substantially present inside the container body. Thus when the container body is opened after the tissue piece is cryopreserved therein, it does not occur that the tissue piece scatters owing to the vaporization of the cooling medium.
   The embodiment of the collected living tissue cryopreserving appliance of the present invention may have the construction described below.
(2) In the collected living tissue cryopreserving appliance according to the above-described (1), the peripheral edge of the container holding part of the container holding member is subjected to chamfering or rounding treatment so that the container holding part does not have an edge which may damage the accommodating bag.
(3) In the collected living tissue cryopreserving appliance according to the above-described (1) or (2), the resin transparent accommodating bag has the outer layer made of the resin resistant to the liquid nitrogen and the heat-sealable inner layer.
(4) In the collected living tissue cryopreserving appliance according to any one of the above-described (1) through (3), the container holding member has the long and narrow holding member body, the container holding part provided at the lower side of the holding member body, and the cooling medium circulating open portion.
(5) In the collected living tissue cryopreserving appliance according to any one of the above-described (1) through (4), the transparent container body has the container body side threaded engaging portion provided at the open portion thereof, and the lid member has the lid member side threaded engaging portion engageable with the container body side threaded engaging portion.
(6) In the collected living tissue cryopreserving appliance according to any one of the above-described (1) through (5), in the state where the lid member is mounted on the container body and the open portion of the container body is sealed with the lid member, the front end of the tissue piece placing member does not contact the bottom surface of the container body.
(7) In the collected living tissue cryopreserving appliance according to any one of the above-described (1) through (6), the tissue piece placing member has the front-end bent portion.
(8) In the collected living tissue cryopreserving appliance according to any one of the above-described (1) through (7), the fixing member of the lid member has the holding member for holding the end portion of the tissue piece placing member and the fixing tool for fixing the holding member to the inner surface of the lid body part.
(9) In the collected living tissue cryopreserving appliance according to any one of the above-described (1) through (8), the tissue piece placing member is formed of the metallic plate-shaped member extended at the predetermined length.
(10) In the collected living tissue cryopreserving appliance according to any one of the above-described (1) through (9), the tissue piece placing member has a large number of holes.
(11) In the collected living tissue cryopreserving appliance according to any one of the above-described (1) through (10), the collected living tissue cryopreserving appliance is the ovarian tissue piece cryopreserving appliance or the liver tissue piece cryopreserving appliance.
   The tissue piece cryopreserving method of the present invention is carried out as described below.
(12) The tissue piece cryopreserving method is carried out by using the collected living tissue cryopreserving appliance according to any one of the above-described (1) through (11). The tissue piece cryopreserving method includes the tissue piece immersing step of immersing the tissue piece collected from the living tissue in the vitrification solution; the tissue piece placing step of taking out the tissue piece from the vitrification solution and placing the tissue piece on the tissue piece placing member of the lid member; the tissue piece sealing step of inserting the tissue piece placing member on which the tissue piece has been placed into the transparent container body without bringing the tissue piece placing member into contact with the cooling medium, mounting the lid member on the container body, and sealing the collected tissue accommodating container; the accommodating bag sealing step of accommodating the sealed collected tissue accommodating container inside the accommodating bag and thereafter heat-sealing the open portion of the accommodating bag; the container body mounting step of mounting the accommodating bag which has accommodated the collected tissue accommodating container therein and has been sealed on the container holding part of the container holding member; and the accommodating step of accommodating the container holding member holding the packaged container body inside the cooling medium tank.
   According to the tissue piece cryopreserving method of the present invention, it is possible to easily and securely perform an operation of freezing the collected tissue piece. Because the collected living tissue cryopreserving appliance is used, during the cryopreservation of the tissue piece, the cooling medium does not flow into the collected tissue accommodating container. Further the collected tissue accommodating container does not contact the cooling medium. Therefore it hardly occurs that the collected tissue accommodating container deteriorates owing to the contact between the collected tissue accommodating container and the cooling medium. The tissue piece does not contact the cooling medium either. Furthermore, the liquid nitrogen is not substantially present inside the container body cryopreserved by carrying out this method. Thus when the container body is opened after the tissue piece is cryopreserved therein, it does not occur that the tissue piece scatters owing to the vaporization of the cooling medium.
   The embodiment of the tissue piece cryopreserving method of the present invention may be as described below.
(13) The tissue piece cryopreserving method according to the above-described (12), which is the ovarian tissue piece cryopreserving method or the liver tissue piece cryopreserving method.

## Claims

1. A collected living tissue cryopreserving appliance (1) for cryopreserving a collected living tissue piece (12) in a sealed state,
wherein said collected living tissue cryopreserving appliance (1) comprises a collected tissue accommodating container (2) capable of accommodating said collected living tissue in a sealed state, a resin transparent accommodating bag (5) which is capable of accommodating said collected tissue accommodating container (2) therein, can be sealed by heat-sealing said resin transparent accommodating bag (5) after accommodating said collected tissue accommodating container (2) therein, and is resistant to liquid nitrogen, and a long and narrow container holding member (6) having a container holding part (62), disposed at a side thereof, for holding said collected tissue accommodating container (2) accommodated inside said accommodating bag (5);
said collected tissue accommodating container (2) comprises a transparent cylindrical container body (3) having an open portion and a lid member (4) which can be removably mounted on said open portion of said transparent container body (3) and can be mounted thereon in an airtight state; and said lid member (4) has a lid body part (42), a tissue piece placing member (41) where said living tissue piece is mountable, a fixing member for fixing said tissue piece placing member (41) to said lid body part (42), and a sealing member (48), formed on an inner surface of said lid body part (42), for sealing said open portion of said transparent container body (3),
said container holding part (62) is axially extended at a predetermined length and formed of two curved portions opposed to each other, said container holding part (62) has a large-diameter portion (62a) formed between upper portions of said two opposed curved portions of said container holding part (62), and an interval between said upper portions of said two opposed curved portions thereof is set larger than other portions thereof and an outer diameter of a lower part of said bottomed cylindrical container body (3), and
a peripheral edge of said container holding part (62) of said container holding member (6) is subjected to chamfering or rounding treatment so that said container holding part (62) does not have an edge which may damage said accommodating bag (5).

2. A collected living tissue cryopreserving appliance (1) according to claim 1, wherein said resin transparent accommodating bag (5) has an outer layer made of resin resistant to liquid nitrogen and a heat-sealable inner layer.

3. A collected living tissue cryopreserving appliance (1) according to any one of claims 1 through 2, wherein said container holding member (6) has a long and narrow holding member body (61), said container holding part (62) provided at a lower side of said holding member body (61), and a cooling medium circulating open portion (63).

4. A collected living tissue cryopreserving appliance (1) according to any one of claims 1 through 3, wherein said transparent container body (3) has a container body side threaded engaging portion (33) provided at said open portion thereof, and said lid member (4) has a lid member side threaded engaging portion (47) engageable with said container body side threaded engaging portion (33).

5. A collected living tissue cryopreserving appliance (1) according to any one of claims 1 through 4, wherein in a state where said lid member (4) is mounted on said container body (3) and said open portion of said container body (3) is sealed with said lid member (4), a front end of said tissue piece placing member (41) does not contact a bottom surface of said container body (3).

6. A collected living tissue cryopreserving appliance (1) according to any one of claims 1 through 5, wherein said tissue piece placing member (41) has a front-end bent portion (46).

7. A collected living tissue cryopreserving appliance (1) according to any one of claims 1 through 6, wherein said fixing member of said lid member (4) has a holding member (43) for holding an end portion of said tissue piece placing member (41) and a fixing tool (44) for fixing said holding member (43) to said inner surface of said lid body part (42).

8. A collected living tissue cryopreserving appliance (1) according to any one of claims 1 through 7, wherein said tissue piece placing member (41) is formed of a metallic plate-shaped member extended at a predetermined length.

9. A collected living tissue cryopreserving appliance (1) according to any one of claims 1 through 8, wherein said tissue piece placing member (41) has a large number of holes.

10. A collected living tissue cryopreserving appliance (1) according to any one of claims 1 through 9, which is an ovarian tissue piece cryopreserving appliance or a liver tissue piece cryopreserving appliance.

11. A tissue piece cryopreserving method using a collected living tissue cryopreserving appliance (1) according to any one of claims 1 through 10, comprising:
a tissue piece immersing step of immersing a tissue piece collected from a living tissue in a vitrification solution;
a tissue piece placing step of taking out said tissue piece from said vitrification solution and placing said tissue piece on said tissue piece placing member (41) of said lid member (4);
a tissue piece sealing step of inserting said tissue piece placing member (41) on which said tissue piece has been placed into said transparent container body (3) without bringing said tissue piece placing member (41) into contact with a cooling medium, mounting said lid member (4) on said container body (3), and sealing said collected tissue accommodating container (2);
an accommodating bag sealing step of accommodating said sealed collected tissue accommodating container (2) inside said accommodating bag (5) and thereafter heat-sealing an open portion of said accommodating bag (5);
a container body mounting step of mounting said accommodating bag (5) which has accommodated said collected tissue accommodating container (2) therein and has been sealed on said container holding part (62) of said container holding member (6); and
an accommodating step of accommodating said container holding member (6) holding said packaged container body (3) inside a cooling medium tank.

12. A tissue piece cryopreserving method according to claim 11, which is an ovarian tissue piece cryopreserving method or a liver tissue piece cryopreserving method.

## Patentansprüche

1. Kryokonservierungsvorrichtung (1) für entnommenes lebendes Gewebe zum Kryokonservieren eines entnommenen lebenden Gewebestücks (12) in einem versiegelten Zustand,
wobei die Kryokonservierungsvorrichtung (1) für entnommenes lebendes Gewebe einen Behälter (2) zum Aufnehmen von entnommenem Gewebe, der in der Lage ist,
das entnommene lebende Gewebe in einem versiegelten Zustand aufzunehmen, einen Beutel (5) zum Aufnehmen aus transparentem Harz, der in der Lage ist, den Behälter (2) zum Aufnehmen von entnommenem Gewebe darin aufzunehmen, durch Heißsiegeln des Beutels (5) zum Aufnehmen aus transparentem Harz nach dem Aufnehmen des Behälters (2) zum Aufnehmen von entnommenem Gewebe darin versiegelt werden kann und gegen flüssigen Stickstoff beständig ist, und ein langes und schmales Behälterhalteelement (6) umfasst, das ein Behälterhalteteil (62) aufweist, welches an einer Seite davon angeordnet ist, zum Halten des Behälters (2) zum Aufnehmen von entnommenem Gewebe, der in dem Beutel (5) zum Aufnehmen aufgenommen ist;
wobei der Behälter (2) zum Aufnehmen von entnommenem Gewebe einen transparenten zylindrischen Behälterkörper (3) umfasst, der einen offenen Abschnitt und ein Deckelelement (4) aufweist, das abnehmbar an dem offenen Abschnitt des transparenten Behälterkörpers (3) befestigt werden kann und das daran in einem luftdichten Zustand befestigt werden kann; und das Deckelelement (4) ein Deckelkörperteil (42), ein Gewebestück-Platzierungselement (41), in dem das lebende Gewebestück anbringbar ist, ein Befestigungselement zum Befestigen des Gewebestück-Platzierungselements (41) an dem Deckelkörperteil (42), und ein Versiegelungselement (48) aufweist, das auf einer Innenfläche des Deckelkörperteils (42) zum Versiegeln des offenen Abschnitts des transparenten Behälterkörpers (3) ausgebildet ist,
wobei das Behälterhalteteil (62) axial um eine vorbestimmte Länge verlängert und aus zwei gekrümmten Abschnitten, die einander gegenüberliegen, gebildet ist, wobei das Behälterhalteteil (62) einen Abschnitt (62a) mit großem Durchmesser aufweist, der zwischen den oberen Abschnitten der beiden gegenüberliegenden gekrümmten Abschnitte des Behälterhalteteils (62) gebildet ist, und ein Zwischenraum zwischen den oberen Abschnitten der beiden gegenüberliegenden gekrümmten Abschnitte davon größer als andere Abschnitte davon und ein Außendurchmesser eines unteren Teils des unteren zylindrischen Behälterkörpers (3) eingestellt ist, und
eine Umfangskante des Behälterhalteteils (62) des Behälterhalteelements (6) einer Anfas- oder Rundungsbehandlung unterzogen wird, sodass das Behälterhalteteil (62) keine Kante aufweist, die den Beutel (5) zum Aufnehmen beschädigen könnte.

2. Kryokonservierungsvorrichtung (1) für entnommenes lebendes Gewebe nach Anspruch 1, wobei der Beutel (5) zum Aufnehmen aus transparentem Harz eine Außenschicht, die aus einem gegen flüssigen Stickstoff beständigen Harz hergestellt ist, und eine heißsiegelbare Innenschicht aufweist.

3. Kryokonservierungsvorrichtung (1) für entnommenes lebendes Gewebe nach einem der Ansprüche 1 bis 2, wobei das Behälterhalteelement (6) einen langen und schmalen Halteelementkörper (61), wobei der Behälterhalteteil (62) an einer Unterseite des Halteelementkörpers (61) vorgesehen ist, und einen offenen Abschnitt (63) für zirkulierendes Kühlmittel aufweist.

4. Kryokonservierungsvorrichtung (1) für entnommenes lebendes Gewebe nach einem der Ansprüche 1 bis 3, wobei der transparente Behälterkörper (3) einen auf der Behälterkörperseite mit Gewinde versehenen Eingriffsabschnitt (33) aufweist, der an dem offenen Abschnitt davon vorgesehen ist, und das Deckelelement (4) einen auf der Deckelelementseite mit Gewinde versehenen Eingriffsabschnitt (47) aufweist, der mit dem auf der Behälterkörperseite mit Gewinde versehenen Eingriffsabschnitt (33) in Eingriff bringbar ist.

5. Kryokonservierungsvorrichtung (1) für entnommenes lebendes Gewebe nach einem der Ansprüche 1 bis 4, wobei in einem Zustand, in dem das Deckelelement (4) an dem Behälterkörper (3) befestigt ist und der offene Abschnitt des Behälterkörpers (3) mit dem Deckelelement (4) versiegelt ist, ein vorderes Ende des Gewebestück-Platzierungselements (41) eine Bodenfläche des Behälterkörpers (3) nicht berührt.

6. Kryokonservierungsvorrichtung (1) für entnommenes lebendes Gewebe nach einem der Ansprüche 1 bis 5, wobei das Gewebestück-Platzierungselement (41) einen gebogenen Vorderendenabschnitt (46) aufweist.

7. Kryokonservierungsvorrichtung (1) für entnommenes lebendes Gewebe nach einem der Ansprüche 1 bis 6, wobei das Befestigungselement des Deckelelements (4) ein Halteelement (43) zum Halten eines Endabschnitts des Gewebestück-Platzierungselements (41) und ein Befestigungswerkzeug (44) zum Befestigen des Halteelements (43) an der Innenfläche des Deckelkörperteils (42) aufweist.

8. Kryokonservierungsvorrichtung (1) für entnommenes lebendes Gewebe nach einem der Ansprüche 1 bis 7, wobei das Gewebestück-Platzierungselement (41) aus einem metallischen plattenförmigen Element gebildet ist, das um eine vorbestimmte Länge verlängert ist.

9. Kryokonservierungsvorrichtung (1) für entnommenes lebendes Gewebe nach einem der Ansprüche 1 bis 8, wobei das Gewebestück-Platzierungselement (41) eine große Anzahl von Löchern aufweist.

10. Kryokonservierungsvorrichtung (1) für entnommenes lebendes Gewebe nach einem der Ansprüche 1 bis 9, die eine Kryokonservierungsvorrichtung für Eierstockgewebestücke oder eine Kryokonservierungsvorrichtung für Lebergewebestücke ist.

11. Kryokonservierungsverfahren für Gewebestücke unter Verwendung einer Kryokonservierungsvorrichtung (1) für entnommenes lebendes Gewebe nach einem der Ansprüche 1 bis 10, umfassend:
einen Gewebestück-Eintauchschritt, bei dem ein aus einem lebenden Gewebe entnommenes Gewebestück in eine Vitrifizierungslösung eingetaucht wird;
einen Gewebestück-Platzierungsschritt, bei dem das Gewebestück aus der Vitrifizierungslösung herausgenommen und das Gewebestück auf das Gewebestück-Platzierungselement (41) des Deckelelements (4) platziert wird;
einen Gewebestück-Versiegelungsschritt, bei dem das Gewebestück-Platzierungselement (41), auf welches das Gewebestück platziert wurde, in den transparenten Behälterkörper (3), ohne das Gewebestück-Platzierungselement (41) mit einem Kühlmedium in Kontakt zu bringen, eingeführt, das Deckelelement (4) auf dem Behälterkörper (3) befestigt und der Behälter (2) zum Aufnehmen des entnommenen Gewebes versiegelt wird;
einen Schritt zum Versiegeln des Beutels zum Aufnehmen für das Aufnehmen des versiegelten Behälters (2) zum Aufnehmen von entnommenem Gewebe in den Beutel (5) zum Aufnehmen und anschließendes Heißversiegeln eines offenen Abschnitts des Beutels (5) zum Aufnehmen;
einen Befestigungsschritt für den Behälterkörper zum Befestigen des Beutels (5) zum Aufnehmen, der den Behälter (2) zum Aufnehmen von entnommenem Gewebe darin aufgenommen hat und der an dem Behälterhalteteil (62) des Behälterhalteelements (6) versiegelt wurde; und
einen Aufnahmeschritt zum Aufnehmen des Behälterhalteelements (6), das den verpackten Behälterkörper (3) innerhalb eines Kühlmitteltanks hält.

12. Kryokonservierungsverfahren für Gewebestücke nach Anspruch 11, das ein Kryokonservierungsverfahren für Eierstockgewebestücke oder ein Kryokonservierungsverfahren für Lebergewebestücke ist.

## Revendications

1. Dispositif (1) de cryopréservation de tissu vivant collecté pour la cryopréservation d'un fragment de tissu vivant collecté (12) dans un état hermétiquement fermé,
dans lequel ledit dispositif (1) de cryopréservation de tissu vivant collecté comprend un récipient de stockage de tissu collecté (2) pouvant stocker ledit tissu vivant collecté dans un état hermétiquement fermé, un sac de stockage transparent en résine (5) qui peut loger ledit récipient de stockage de tissu collecté (2), peut être scellé en thermosoudant ledit sac de stockage transparent en résine (5) après y avoir placé ledit récipient de stockage de tissu collecté (2), et est résistant à l'azote liquide, et un élément de support (6) de récipient long et étroit ayant une partie de support de récipient (62), disposé au niveau d'un côté, pour supporter ledit récipient de stockage de tissu collecté (2) logé à l'intérieur dudit sac de stockage (5) ;
ledit récipient de stockage de tissu collecté (2) comprend un corps de récipient cylindrique transparent (3) ayant une partie ouverte et un élément de couvercle (4) qui peut être monté de façon amovible sur ladite partie ouverte dudit corps de récipient transparent (3) et peut être monté sur celle-ci de façon étanche à l'air ; et ledit élément de couvercle (4) comprend une partie de corps de couvercle (42), un élément de positionnement de fragment de tissu (41) où ledit fragment de tissu vivant peut être monté, un élément de fixation pour fixer ledit élément de positionnement de fragment de tissu (41) à ladite partie de corps de couvercle (42), et un élément d'étanchéité (48), formé sur une surface intérieure de ladite partie de corps de couvercle (42), pour fermer hermétiquement ladite partie ouverte dudit corps de récipient transparent (3),
ladite partie de support de récipient (62) s'étend axialement sur une longueur prédéterminée et est formée de deux parties courbées se faisant face, ladite partie de support de récipient (62) a une partie de grand diamètre (62a) formée entre des parties supérieures desdites deux parties courbées opposées de ladite partie de support de récipient (62), et un intervalle entre lesdites parties supérieures desdites deux parties courbées opposées de celle-ci est établi pour être plus grand que ses autres parties et un diamètre extérieur d'une partie inférieure dudit corps de récipient cylindrique muni d'un fond (3), et
un bord périphérique de ladite partie de support de récipient (62) dudit élément de support de récipient (6) est soumis à un traitement de chanfreinage ou arrondissage de sorte que ladite partie de support de récipient (62) n'a pas de bord qui puisse endommager ledit sac de stockage (5).

2. Dispositif (1) de cryopréservation de tissu vivant collecté selon la revendication 1, dans lequel ledit sac de stockage transparent en résine (5) comprend une couche extérieure faite de résine résistant à l'azote liquide et une couche intérieure thermosoudable.

3. Dispositif (1) de cryopréservation de tissu vivant collecté selon l'une quelconque des revendications 1 à 2, dans lequel ledit élément de support de récipient (6) a un corps d'élément de support long et étroit (61), ladite partie de support de récipient (62) prévue au niveau d'un côté inférieur dudit corps d'élément de support (61), et une partie ouverte (63) de circulation de produit de refroidissement.

4. Dispositif (1) de cryopréservation de tissu vivant collecté selon l'une quelconque des revendications 1 à 3, dans lequel ledit corps de récipient transparent (3) a une partie d'engagement filetée (33) côté corps de récipient prévue au niveau de son extrémité ouverte, et ledit élément de couvercle (4) a une partie d'engagement filetée (47) côté élément de couvercle pouvant venir en prise avec ladite partie d'engagement filetée (33) côté corps de récipient.

5. Dispositif (1) de cryopréservation de tissu vivant collecté selon l'une quelconque des revendications 1 à 4, dans lequel dans un état où ledit élément de couvercle (4) est monté sur ledit corps de récipient (3) et où ladite partie ouverte dudit corps de récipient (3) est fermée hermétiquement par ledit élément de couvercle (4), une extrémité avant dudit élément de positionnement de fragment de tissu (41) ne vient pas en contact avec une surface inférieure dudit corps de récipient (3).

6. Dispositif (1) de cryopréservation de tissu vivant collecté selon l'une quelconque des revendications 1 à 5, dans lequel ledit élément de positionnement de fragment de tissu (41) a une partie d'extrémité avant courbée (46).

7. Dispositif (1) de cryopréservation de tissu vivant collecté selon l'une quelconque des revendications 1 à 6, dans lequel ledit élément de fixation dudit élément de couvercle (4) a un élément de support (43) pour supporter une partie d'extrémité dudit élément de positionnement de fragment de tissu (41) et un outil de fixation (44) pour fixer ledit élément support (43) à ladite surface intérieure de ladite partie de corps de couvercle (42).

8. Dispositif (1) de cryopréservation de tissu vivant collecté selon l'une quelconque des revendications 1 à 7, dans lequel ledit élément de positionnement de fragment de tissu (41) est formé d'un élément en forme de plaque métallique qui s'étend sur une longueur prédéterminée.

9. Dispositif (1) de cryopréservation de tissu vivant collecté selon l'une quelconque des revendications 1 à 8, dans lequel ledit élément de positionnement de fragment de tissu (41) a un grand nombre d'orifices.

10. Dispositif (1) de cryopréservation de tissu vivant collecté selon l'une quelconque des revendications 1 à 9, qui est un dispositif de cryopréservation de fragment de tissu ovarien ou un dispositif de cryopréservation de fragment de tissu du foie.

11. Procédé de cryopréservation de fragment de tissu à l'aide d'un dispositif (1) de cryopréservation de tissu vivant collecté selon l'une quelconque des revendications 1 à 10, comprenant :
une étape d'immersion de fragment de tissu consistant à immerger un fragment de tissu prélevé sur un tissu humain dans une solution de vitrification ;
une étape de mise en place de fragment de tissu consistant à sortir ledit fragment de tissu de ladite solution de vitrification et à placer ledit fragment de tissu sur ledit élément de positionnement de fragment de tissu (41) dudit élément de couvercle (4) ;
une étape de scellement de fragment de tissu consistant à insérer ledit élément de positionnement de fragment de tissu (41) sur lequel ledit fragment de tissu a été placé dans ledit corps de récipient transparent (3) sans amener ledit élément de positionnement de fragment de tissu (41) en contact avec un produit de refroidissement, à monter ledit élément de couvercle (4) sur ledit corps de récipient (3), et à fermer hermétiquement ledit récipient de stockage de tissu collecté (2) ;
une étape de scellement de sac de stockage consistant à placer ledit récipient de stockage de tissu collecté (2) à l'intérieur dudit sac de stockage (5), puis à thermosouder une partie ouverte dudit sac de stockage (5) ;
une étape de montage de corps de récipient consistant à monter ledit sac de stockage (5) dans lequel a été placé ledit récipient de stockage de tissu collecté (2) et qui a été scellé sur ladite partie de support de récipient (62) dudit élément de support de récipient (6) ; et
une étape de mise en place consistant à placer ledit élément de support de récipient (6) supportant ledit corps de récipient enveloppé (3) à l'intérieur d'un réservoir de produit de refroidissement.

12. Procédé de cryopréservation de fragment de tissu selon la revendication 11, qui est un procédé de cryopréservation de fragment de tissu ovarien ou un procédé de cryopréservation de fragment de tissu du foie.
